# EUROPEAN PATENT SPECIFICATION

(11) **EP 3 790 477 B1**
(45) Date of publication and mention of the grant of the patent: **09.02.2022**
(21) Application number: 19729834.2
(22) Date of filing: 08.05.2019
(51) Int. Cl.: A61B 17/17, A61B 34/20

(54) **SURGICAL DEVICE OF REPERE**
CHIRURGISCHE MARKIERUNGSVORRICHTUNG
DISPOSITIF CHIRURGICAL DE REPÈRE

(30) Priority: 10.05.2018 IT 201800005244
(43) Date of publication of application: 17.03.2021
(73) Proprietor: MT Ortho S.r.l., 95025 Aci Sant'Antonio (IT)
(72) Inventor: NICOLETTI, Giovanni Federico, 95021 Aci Castello CT (IT)
(74) Representative: Manna, Sara
(86) International application number: PCT/IB2019/053764
(87) International publication number: WO 2019/215627

(56) References cited:
- US-A- 4 907 577
- US-A1- 2010 023 018
- US-A1- 2011 218 546
- US-A1- 2017 348 061

## Description

### Technical field of the invention

The present invention relates to a surgical device of repere, apt to provide a visual spatial and radiologically identifiable reference of the position of a vertebral spinous process.

The device the present invention relates to is meant in particular as help to a surgical operator in performing operations of the spinal column. In particular, the device can be advantageously used during the carrying-out of operations of arthrodesis performed with the help of intraoperative tomographs (for example O-ARM).

### Background

Thanks to the technological development which has occurred during the most recent years, the spinal surgery can avail of the intraoperative use of computerized devices (tomographs) configured to provide 3D images of the treated spinal districts.

In particular, such tools help the surgeon during operations which provide the implantation of screws of vertebral stabilization systems. Such screws have to be implanted according to penetration trajectories and positions predetermined by the surgeon itself.

The correct positioning of the screws is a crucial factor for the success of the operation, as should these be placed according to trajectories different from the predetermined ones, this would involve the risk of serious lesions of the column nervous structures, as well as lesions of vital veins and arteries and of the same bone structures.

The computerized devices known in the state of art allow to some extent to monitor the positioning of such screws to verify that it is correct, that is it meets the criteria predetermined by the surgical operator.

To this purpose, the above-mentioned devices generally are interfaced to neuro-navigation systems (for example of the O-ARM / Navigator type) which allow the surgeon to follow the route of the screws of the stabilization systems inserted into the vertebrae in the three space sizes.

By means of the help of such computerized control apparatuses the risk that the screws arrange according to not correct routes is reduced to some extent.

However, errors in detecting the trajectories of the above-mentioned screws performed by means of the devices of known type are possible. Such possibility is inherent in the fact that the above-mentioned computerized devices are calibrated with respect to reference systems *('reference frame')* which are positioned at the beginning of the operation. The positioning of such reference systems affects strongly the accuracy of the detection of the screws' position.

It is recognized that the above-mentioned reference devices can be subjected to dislocations during the operation, which, even if minimum, are sufficient to induce the surgeon in very serious errors.

The only way which can be used today to avoid such errors is to perform, during the operation, several checks of the calibration of the computerized devices which provide the 3D images of the spinal districts under surgical treatment, with periodical radiological acquisition of images during the operation.

This inevitably involves an increase in surgical time, affecting both the surgeon and the patient.

Moreover, such repeated calibration checks involve for the patients and for the whole involved medical personnel a dangerous overexposure to radiations.

US20110218546A1 describes a surgical device comprising a fixed portion intended to be connected to a bone of a patient, a mobile portion with respect to said fixed portion and position indicating means, in the form of a tracing element apt to be detected by a navigation system.

US20170348061A1 describes a surgical robot associated to reference means which can be applied to the body district of a patient, comprising an array having four markers apt to assist the spatial coordination of the robot itself with respect to the patient.

### Summary of the invention

The technical problem placed and solved by the present invention is then to provide a surgical device allowing to obviate the drawbacks mentioned above with reference to the known art.

The above-mentioned drawbacks are solved by a surgical device of repère according to claim 1.

Preferred features of the present invention are set forth in the depending claims.

The present invention provides a device suitable to provide a fixed visual reference point which can be identified by the tomographs, helping the surgeon to perform operations of the spinal column.

To this purpose, the device comprises a skin contact element configured for abutting on the skin of the patient, at the spinous process of a vertebra, and coupling means configured to penetrate and insert within the above-mentioned spinous process.

The coupling means is configured to implement a connection with the spinous process which is apt to remain fixed during the whole duration of the operation. The coupling means has a main development direction, or longitudinal development direction, which coincides with the preferential direction of insertion of the same in the spinous process.

The skin contact element in turn is connected to handling means of the device itself, and above all to position indicating means.

The position indicating means is configured to indicate the penetration point of the coupling means in the spinous process. Therefore, when the device is in use, the position indicating means is arranged along the main development direction, or longitudinal development direction, of the coupling means.

The device according to the present invention allows to provide the surgeon a point of repere, in other words, a spatial visual reference of a particular anatomic element, which keeps constant during the carrying-out of the operation.

In particular, the use of the device can be provided in addition to the reference systems of traditional type, associated to the computerized devices which provide 3D images of the involved spinal districts.

Advantageously, the device of the invention allows to be able to check, without interruptions, in real time, the correct calibration of neuro-navigation systems during the carrying-out of surgical operations of the spinal column.

Still more advantageously, the continuous monitoring allowed by means of the device does not provide the need for new radiological acquisitions. Therefore, the medical personnel and the patient are not exposed to additional doses of radiations.

According to a particularly preferred embodiment of the invention, the above-mentioned coupling means comprises an oblong engagement element, shaped like a screw, provided with external thread. In particular, such engagement element has a free end provided with a sharp point, which favours the penetration of the coupling means within the vertebral spinous process.

The particular screw-like shape of the engagement element allows to implement a fastening mode of the device for screwing within the spinous process, to implement a threaded coupling (at least regarding the portion of the device of the invention).

Such coupling, even if reversible, is characterized by a high precision in mutual position of the coupled bodies (spinous device and process), which keeps constant in time. In the specific case, the coupling configuration makes it very improbable, if not impossible, even the minimum dislocation of the device with respect to its seat of insertion in the spinous process.

Still, according to particularly advantageous variants of the invention, the device is made partially or wholly of radiopaque material, that is material which does not make the X rays to pass through it.

Advantageously, thanks to the use of the device according to the present invention, the surgeon can control the exact calibration of the tools in few seconds, exclusively by resting the *'passive planner'* or the *'pack needle'* on the vertex of the device according to the present invention.

Other advantages, features and use modes of the present invention will result evident from the following detailed description of some embodiments, shown by way of example and not for limitative purposes.

### Brief description of the figures

The enclosed Figures will be referred to, wherein:
- Figure 1 shows a front perspective view of a first preferred embodiment of a surgical device according to the present invention;
- Figure 2 shows an enlargement of an upper portion of the device of Figure 1;
- Figures 3A to 3C show, respectively in sequence, perspective views of application phases of the device of Figure 1 to a spinous process, respectively a positioning phase, a penetration phase and a phase for connecting position indicating means to the main body of the device;
- Figure 4 shows an enlarged view, in exploded configuration, of a detail of Figure 3C, wherein an upper portion of the device of Figure 1 can be seen, including position indicating means.

The above-mentioned Figures are to be meant exclusively by way of example and not for limitative purposes. In particular, the lengths, thicknesses and mutual proportions between the elements represented therein can have different - larger or smaller - size parameters - with respect to what described in the following section, by way of representation clarity.

### Detailed description of preferred embodiments

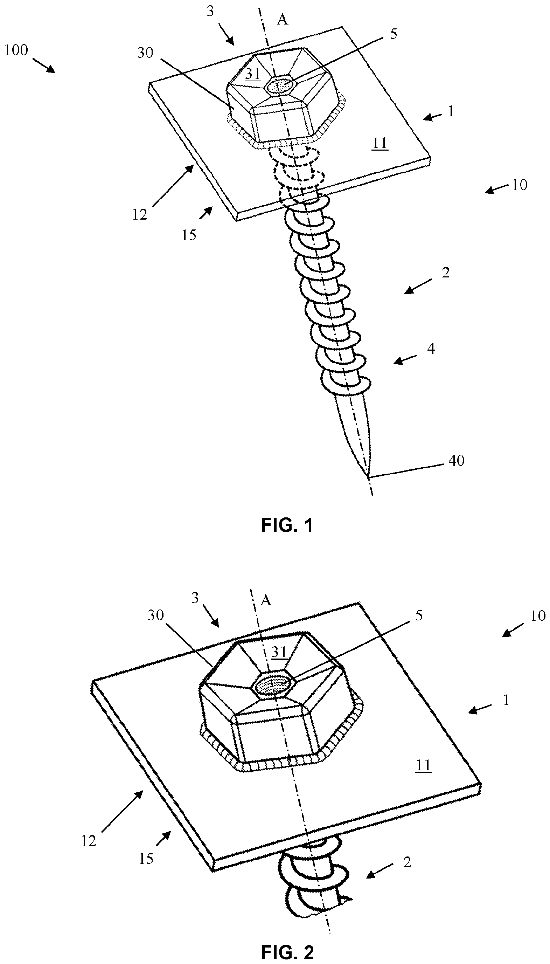

By firstly referring to Figures 1 and 2, a preferred embodiment of a surgical device according to the present invention is designated as a whole with 100. In particular, the device of the invention is suitable to provide a visual spatial reference for helping to carry out spinal operations, such as operations of arthrodesis.

The device 100 substantially comprises a main body 10, which, in turn, comprises a skin contact element 1, coupling means 2 with the spinous process of the patient and handling means 3.

The skin contact element 1 is configured to abut on the skin overlying a vertebral spinous process, at least at its own abutment surface 15 suitably configured to the purpose.

To this purpose, according to the preferred embodiment shown in the enclosed Figures, the skin contact element 1 preferably has a plate-like shape, comprising a lower face 12 and an upper face 11, opposite to each other. Preferably, the skin contact element 1 has a quadrangular or square shape in plan. According to the preferred embodiment shown in the enclosed Figures, the device 100 comprises a skin contact element 1 constituted by a square-plan plate wherein each side is about 1.5 cm, whereas the plate thickness is equal to about 3 mm.

The lower face 12 comprises the above-mentioned abutment surface 15, shaped to abut - directly or indirectly, that is after mediation of one or more additional elements - on the skin overlaying the vertebral spinous process of the patient. The abutment surface 15 preferably has a quadrangular or square profile in plan and still more preferably has a substantially flat or planar development.

Moreover, the main body 10 comprises handling means 3 connected to the skin contact element 1, configured to allow the surgeon to be able to adjust the coupling between the device 100 itself and the spinous process. Moreover, the handling means 3 eases the operator in handling the device 100. Such means 3 can include a head element 30, for example shaped like a hexagon nut (as visible in Figure 2), configured to be able to engage with a tightening wrench or other equivalent means.

Such means 3, with particular reference to the head element 30, preferably is arranged at the upper face 11 of the skin contact element 1, in particular it is opposed to the abutment surface 15, so as to remain external to the spinous process and always to allow the operator to adjust and extract the device 100, during and after the surgical operation.

In particular, the head element 30 is connected to the upper face 11 in a permanent manner, for example by means of welding, to guarantee a firm and resistant connection.

The head element 30 preferably comprises a concave external upper surface 31, in particular the peripheral edges of the external upper surface 31 result to be more projecting - with reference to the skin contact element 1 - of the central portion of the same external upper surface 31, and therefore farer from the upper face 11. According to the preferred embodiment shown in the enclosed Figures, the maximum thickness of the head element 30, that is the distance between the peripheral edges of the external upper surface 31 and the upper face 11, is equal to about 5 mm. Such distance is of course reduced at the central portion of the upper face 11 should the latter have a concave configuration, wherein the concavity is maximum indeed at the above-mentioned central portion.

As said before, the main body 10 further comprises coupling means 2 with the spinous process of the patient, which is connected to the skin contact element 1 preferably at the lower face 12, or still more preferably at the abutment surface 15.

The coupling means 2 is shaped to penetrate the external surface of the spinous process and to insert at least partially inside thereof. To this purpose, the coupling means 2 comprises at least a protruding element 4, preferably having an overall oblong shape or stem-like shape, apt to penetrate the spinous process.

The coupling means 2, and in particular at least a protruding element 4, has a main development axis, or longitudinal development axis, which preferably coincides with its own axis of symmetry A, still more preferably an axis of central symmetry A.

Hereinafter, the main development or longitudinal axis of the means 2 or of the protruding element 4 will be referred to as main development or longitudinal direction.

In order to ease the penetration of at least a protruding element 4 within the spinous process, such protruding element 4 preferably is provided with a free terminal end 40, opposed to the abutment surface 15, having a sharp point. Such point, which has no threads, develops along the axis A for a length preferably comprised between about 3 and 8 mm.

With the purpose of increasing the contact area of the coupling means 2 with the spinous process, while maintaining reduced the overall sizes, such at least a protruding element 4 comprises preferably a threaded external surface. According to such embodiment variant, shown in the enclosed Figures, the at least a protruding element 4 is substantially comparable to the stem of a screw and it has in particular a circular cross section, having constant diameter and preferably equal to 2 mm. By way of example, the threaded surface extends along the axis A for a length comprised between about 1 and 12 cm along the above-mentioned main development direction, proceeding from the abutment surface 15 towards the free terminal end.

The length of the sharp point 40 and of the external threaded surface 4, considered along the axis A, are variable compared to the height of the involved spinous process and to the thickness of the adipose layer interposed between apex of the spinous process and cutaneous surface. Such measures can be determined by analysing the X-ray diagnostic images, CAT, Magnetic Resonance or other diagnostic system.

Moreover, preferably, the abutment surface 15 is arranged to surround at least partially the coupling means 2 in order to favour a stable connection with the spinous process and to avoid unwished motions of the device 100 once implanted.

In the particular embodiment variant shown in the enclosed Figures, which provides the presence of a single protruding element 4, the latter has a main development direction according to its own axis of symmetry A, which in particular coincides with an axis of central symmetry of the device 100 considered as a whole, at least according to a configuration wherein the skin contact element 1 and the handling means 3 are centred with respect to such axis A. In other words, the device 100 can be symmetric with respect to the axis A.

In particular, the axis of symmetry A develops along a longitudinal direction, substantially orthogonal with respect to the abutment surface 15. The axis of symmetry A, or generally the main development direction - or longitudinal development direction - of at least a protruding element 4, defines the preferential direction of insertion of the coupling means 2 in the spinous process.

Still, the device 100 comprises position indicating means 60, arranged to designate the penetration point of the coupling means 2 in the spinous process.

The position indicating means 60 is configured to designate the penetration point of the coupling means 2, and in particular of the protruding element 4 in the spinous process. Therefore, when it is connected to the main body 10, the position indicating means 60 is arranged along the main development direction, or longitudinal development direction, of the coupling means 2 or of the protruding element 4, the latter being the preferential direction of insertion of the same in the spinous process.

In the particular embodiment variant shown in the enclosed Figures, providing the presence of a single protruding element 4, the position indicating means 60 is configured to connect to the main body 10 at the axis of symmetry A of such protruding element 4, which coincides with the main development or longitudinal direction of the protruding element 4. Preferably, the means 60 is connected or can be connected to the main body 10 in centred position with respect to the latter, and in each case so as to remain well visible and still external to the back of the patient, even after the connection of the device 100 to the spinous process, to implement a point of repere.

According to the preferred embodiment illustrated in the enclosed Figures, at a central portion of the head element 30, or better at a central portion of the external upper surface 31, at least a seat is obtained, configured to engage with, or receive, the position indicating means 60. Such seat is preferably shaped to implement one hole 5. The functionality assigned to the hole 5 is that of allowing the reversible engagement with respective position indicating means 60.

According to what already described, the hole 5 is implemented at the main development or longitudinal direction of the protruding element 4, which corresponds to the axis of symmetry A shown in the enclosed Figures.

In particular, the position indicating means 60 can include a plug element or cap 6 apt to act as point of repere, configured to engage with the hole 5.

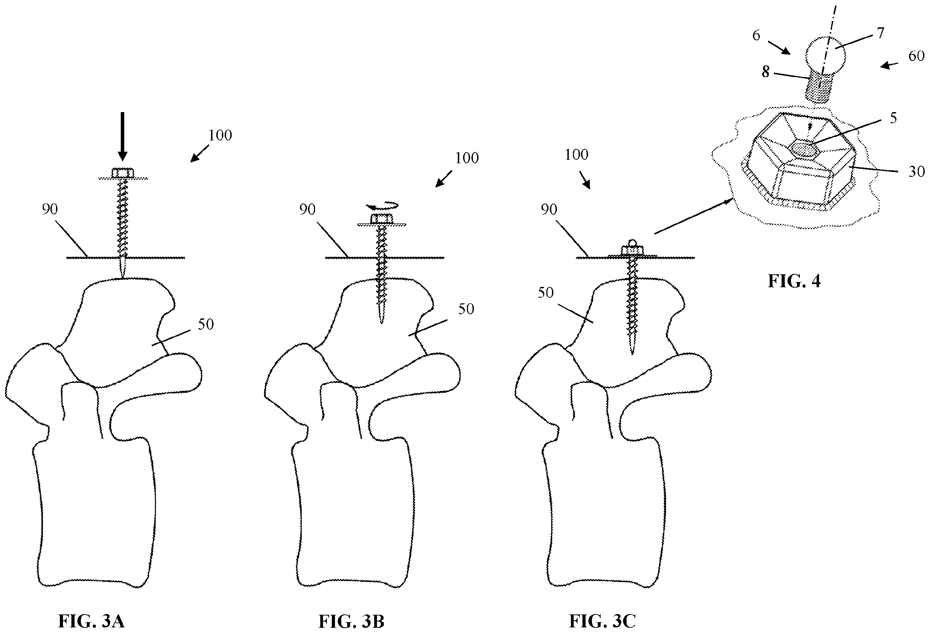

Figure 4 shows a preferred embodiment of such cap 6. The cap 6 has a head portion 7 and a stem portion 8. Preferably, the head portion 7 has substantially spherical geometry, preferably with diameter equal to about 2 mm.

The stem portion 8 is shaped to engage with the hole 5. In particular, the cap 6 can be coupled with the main body 10 by inserting said stem portion within the hole 5. With the purpose of implementing a reversible but stable coupling between the cap 6 and the main body 10 (in particular between the cap 6 and the head element 30), the hole 5 can have an inner thread and the stem portion 8 can have a respective external thread. The stem portion 8 has a length preferably equal to 5mm, which coincides with a respective minimum depth of the hole 5.

Once the cap 6 is connected to the main body 10, in turn inserted into a spinous process, the head portion 7 implements for the surgeon a visual reference point - point of repère - helping him/her during the carrying-out of spinal interventions.

Alternatively, modes for connecting at least a cap 6 to the main body 10, different from the above-described one, can be provided, wherein the cap 6 is fastened reversibly to the main body 10 or directly to the skin contact element 1.

With reference to what already described, the external upper surface face 31 of the head element 30 is preferably concave to allow to beat - for example with a surgical hammer - on the above-mentioned external upper surface 31, with the purpose of implementing the penetration of the main body 10 in the spinous process, without the beating involves the hole 5 and runs the risk of jeopardizing the capability of engaging with the cap 6, for example by ruining the thread.

The material the device 100 is made of, preferably is a radiopaque material, still more preferably high-tensile and bio-compatible material, for example comprising at least partially steel and/or titanium.

With reference to Figures 3A, 3B and 3C, the application phases of a preferred embodiment of the device 100 to the vertebral spinous process 50 of a patient are described hereinafter. The phases are numbered according to the chronological order for performing the same.

The result obtained by applying the device to the patient is to provide to the surgeon a visual indication of a spatial reference point identifiable by the tomograph, which remains fixed for the whole operation duration, in order to monitor the correct calibration of computerized systems, for example of O-ARM / Navigator type.

### Phase 1 - positioning (Figure 3A)

The already anaesthetized patient is positioned on the operating table. The surgical device 100 according to the present invention is put near the skin 90 at a predetermined spinous process 50 of the patient. Such spinous process 50 is different from the one which will house the traditional reference system.

In particular, the sharp end 40, by perforating the skin 90, is rested upon the apex of the predetermined spinous process 50, exactly in the point wherein it should penetrate the back of the patient.

The positioning of the sharp end 40 can be performed by availing of the control of a computerized monitoring system, for example of the O-ARM system, used in this 2D phase.

### Phase 2 - penetration (Figure 3B)

The surgeon exerts pressing actions on the device 100 to implement the penetration of the sharp point 40 of the coupling means 2 in the predetermined spinous process 50, in particular by beating with a surgical hammer on the upper surface 31 of the head element 30.

### Phase 3 - tightening (Figures 3B and 3C)

When the sharp point 40 is inserted into the spinous process 50, one proceeds with screwing and tightening precisely the device 100 by means of wrench or another tightening tool. For example, a hexagon wrench can be used, referred to a head element 30 shaped like a hexagon nut.

The tightening is considered completed when the abutment surface 15 enters in contact with the skin 90 of the patient, or better it abuts thereon.

### Phase 4 - reference assembly (Figure 4)

Once completed the penetration and tightening phases, the position indicating means 60 is connected to the main body 10. With specific reference to the embodiment comprising the cap 6, the stem portion 8 is inserted into the hole 5, in centred position with respect to the head element 30 and the main body 10, and it is screwed up for the whole length of its thread within the hole 5.

Once ended the positioning of the cap 6, which acts as spatial reference or better as point of repere, one can proceed with the acquisition of the 3D images of the involved spinal district.

The present invention has been sofar described with reference to preferred embodiments. It is to be meant that other embodiments belonging to the same inventive core may exist, as defined by the protective scope of the herebelow reported claims.

## Claims

1. A surgical device (100) of repère configured to be connected to a vertebral spinous process (50) of a patient, said surgical device (100) having a main body (10) which in turn comprises:
- a skin contact element (1) having a lower face (12) and an upper face (11) opposite to each other, wherein said lower face (12) comprises an abutment surface (15) shaped to abut on the skin (90) of the patient at the vertebral spinous process (50);
- handling means (3) connected to said skin contact element (1), configured to allow adjustment of the coupling between said device (100) and the vertebral spinous process (50), said handling means (3) comprising a head element (30);
- coupling means (2) with the vertebral spinous process (50), connected to said skin contact element (1) at said abutment surface (15) and shaped to penetrate the external surface of the vertebral spinous process (50) and configured to be inserted at least partially inside thereof, said coupling means (2) comprising at least one protruding element (4) with respect to said abutment surface (15) having a longitudinal direction (A); wherein said surgical device (100) further comprises position indicating means (60) configured to be connected to said main body (10),
**characterized in that** said position indicating means (60), is connected to said main body (10) along the longitudinal direction (A) of said protruding element (4), the latter being an insertion preferential direction of said protruding element (4) in the vertebral spinous process (50), so that said position indicating means (60) indicates the penetration point of said protruding element (4) in the vertebral spinous process (50).

2. The surgical device (100) according to claim 1, wherein said skin contact element (1) has a plate-shaped conformation, said conformation being preferably quadrangular or square in plan.

3. The surgical device (100) according to claim 1 or 2, wherein said abutment surface (15) has a substantially flat or planar development.

4. The surgical device (100) according to anyone of the preceding claims, wherein said head element (30) is connected to said upper face (11), preferably in a permanent manner.

5. The surgical device (100) according to anyone of the preceding claims, wherein said head element (30) is shaped like a hexagonal nut.

6. The surgical device (100) according to anyone of the preceding claims, wherein said head element (30) comprises a concave external upper surface (31).

7. The surgical device (100) according to anyone of the preceding claims, wherein said at least a protruding element (4) has an overall oblong or stem-like conformation.

8. The surgical device (100) according to anyone of the preceding claims, wherein said at least a protruding element (4) extends according to a 2. longitudinal direction (A) substantially orthogonal with respect to said abutment surface (15).

9. The surgical device (100) according to anyone of the preceding claims, wherein said at least a protruding element (4) has a free terminal end (40) having a sharp point.

10. The surgical device (100) according to anyone of the preceding claims, wherein said at least a protruding element (4) comprises a threaded external surface, being substantially configured as a screw stem.

11. The surgical device (100) according to anyone of the preceding claims, comprising a single protruding element (4) having an axis of symmetry coinciding with said longitudinal direction (A).

12. The surgical device (100) according to anyone of the preceding claims, wherein said position indicating means (60) is arranged in centred position with respect to said main body (10).

13. The surgical device (100) according to anyone of the preceding claims, having an axis of central symmetry which coincides with said longitudinal direction (A) and defines a preferential direction of insertion of said coupling means (2) in the vertebral spinous process (50).

14. The surgical device (100) according to anyone of the preceding claims, wherein said position indicating means (60) comprises a plug element (6) and said head element (30) comprises at least one hole (5) obtained at its own central portion and at said longitudinal direction (A), said plug element (6) being configured to engage reversibly with said at least one hole (5).

## Patentansprüche

1. Chirurgische Markierungsvorrichtung (100), die so ausgestaltet ist, dass sie mit einem Wirbeldornfortsatz (50) eines Patienten verbunden wird,
wobei die chirurgische Vorrichtung (100) einen Hauptkörper (10) hat, welcher wiederum umfasst:
- ein Hautkontaktelement (1) mit einer unteren Fläche (12) und einer oberen Fläche (11), die einander gegenüberliegen, wobei die untere Fläche (12) eine Anlagefläche (15) umfasst, die so geformt ist, dass sie an der Haut (90) des Patienten an dem Wirbeldornfortsatz (50) anliegt;
- ein Handhabungsmittel (3), das mit dem Hautkontaktelement (1) verbunden ist und das so ausgestaltet ist, dass es ein Einstellen der Verbindung zwischen der Vorrichtung (100) und dem Wirbeldornfortsatz (50) ermöglicht, wobei das Handhabungsmittel (3) ein Kopfelement (30) umfasst;
- ein Kopplungsmittel (2) mit dem Wirbeldornfortsatz (50), das mit dem Hautkontaktelement (1) an der Anlagefläche (15) verbunden ist und so geformt ist, dass es die Außenfläche des Wirbeldornfortsatzes (50) durchdringt und dazu ausgestaltet ist, zumindest teilweise in dessen Inneres eingeführt zu werden, wobei das Kopplungsmittel (2) wenigstens ein vorstehendes Element (4) bezüglich der Anlagefläche (15) umfasst, das eine Längsrichtung (A) hat;
wobei die chirurgische Vorrichtung (100) ferner ein Positionsanzeigemittel (60) umfasst, das dazu ausgestaltet ist, um mit dem Hauptkörper (10) verbunden zu werden, **dadurch gekennzeichnet, dass** das Positionsanzeigemittel (60) mit dem Hauptkörper (10) entlang der Längsrichtung (A) des vorstehenden Elements (4) verbunden ist, wobei letzteres eine Vorzugseinsetzrichtung des vorstehenden Elements (4) in den Wirbeldornfortsatz (50) ist, so dass das Positionsanzeigemittel (60) den Eintrittspunkt des vorstehenden Elements (4) in den Wirbeldornfortsatz (50) anzeigt.

2. Chirurgische Vorrichtung (100) gemäß Anspruch 1, wobei das Hautkontaktelement (1) eine plattenförmige Ausgestaltung hat, wobei die Ausgestaltung im Grundriss vorzugsweise viereckig oder quadratisch ist.

3. Chirurgische Vorrichtung (100) gemäß Anspruch 1 oder 2, wobei die Anlagefläche (15) einen im Wesentlichen flachen oder ebenen Aufbau hat.

4. Chirurgische Vorrichtung (100) gemäß einem der vorhergehenden Ansprüche, wobei das Kopfelement (30) mit der oberen Fläche (11) verbunden ist, vorzugsweise in einer dauerhaften Weise.

5. Chirurgische Vorrichtung (100) gemäß einem der vorhergehenden Ansprüche, wobei das Kopfelement (30) wie eine Sechskantmutter geformt ist.

6. Chirurgische Vorrichtung (100) gemäß einem der vorhergehenden Ansprüche, wobei das Kopfelement (30) eine konkave externe obere Fläche (31) umfasst.

7. Chirurgische Vorrichtung (100) gemäß einem der vorhergehenden Ansprüche, wobei das wenigstens eine vorstehende Element (4) eine insgesamt längliche oder stilartige Ausgestaltung hat.

8. Chirurgische Vorrichtung (100) gemäß einem der vorhergehenden Ansprüche, wobei das wenigstens eine vorstehende Element (4) sich entlang einer Längsrichtung (A) erstreckt, die im Wesentlichen senkrecht zu der Anlagefläche (15) ist.

9. Chirurgische Vorrichtung (100) gemäß einem der vorhergehenden Ansprüche, wobei das wenigstens eine vorstehende Element (4) ein freies Ende (40) mit einer scharfen Spitze hat.

10. Chirurgische Vorrichtung (100) gemäß einem der vorhergehenden Ansprüche, wobei das wenigstens eine vorstehende Element (4) eine Gewindeoberfläche umfasst, die im Wesentlichen als ein Schraubenschaft ausgestaltet ist.

11. Chirurgische Vorrichtung (100) gemäß einem der vorhergehenden Ansprüche, umfassend ein einzelnes vorstehendes Element (4) mit einer Symmetrieachse, die mit der Längsrichtung (A) zusammenfällt.

12. Chirurgische Vorrichtung (100) gemäß einem der vorhergehenden Ansprüche, wobei das Positionsanzeigemittel (60) bezüglich des Hauptkörpers (10) in einer zentrierten Position angeordnet ist.

13. Chirurgische Vorrichtung (100) gemäß einem der vorhergehenden Ansprüche, umfassend eine zentrale Symmetrieachse, die mit der Längsrichtung (A) zusammenfällt und eine bevorzugte Einsetzrichtung des Kopplungsmittels (2) in den Wirbeldornfortsatz (50) definiert.

14. Chirurgische Vorrichtung (100) gemäß einem der vorhergehenden Ansprüche, wobei das Positionsanzeigemittel (60) ein Steckelement (6) umfasst, und das Kopfelement (30) wenigstens ein Loch (5) umfasst, das an seinem eigenen Zentralabschnitt und in der Längsrichtung (A) liegt, wobei das Steckelement (6) so ausgestaltet ist, dass es reversibel mit dem wenigstens einen Loch (5) in Eingriff kommt.

## Revendications

1. Dispositif chirurgical (100) de repère configuré pour être raccordé à une apophyse épineuse vertébrale (50) d'un patient :
ledit dispositif chirurgical (100) ayant un corps principal (10) qui comprend à son tour :
- un élément de contact cutané (1) ayant une face inférieure (12) et une face supérieure (11) opposées entre elles, dans lequel ladite face inférieure (12) comprend une surface de butée (15) formée pour venir en butée sur la peau (90) du patient au niveau de l'apophyse épineuse vertébrale (50) ;
- un moyen de manipulation (3) raccordé audit élément de contact cutané (1), configuré pour permettre l'ajustement du couplage entre ledit dispositif (100) et l'apophyse épineuse vertébrale (50), ledit moyen de manipulation (3) comprenant un élément de tête (30) ;
- un moyen de couplage (2) avec l'apophyse épineuse vertébrale (50), raccordé audit élément de contact cutané (1) au niveau de ladite surface de butée (15) et formé pour pénétrer dans la surface externe de l'apophyse épineuse vertébrale (50) et configuré pour être inséré au moins partiellement dans son intérieur,
ledit moyen de couplage (2) comprenant au moins un élément en saillie (4) par rapport à ladite surface de butée (15) ayant une direction longitudinale (A) ;
dans lequel ledit dispositif chirurgical (100) comprend en outre un moyen d'indication de position (60) configuré pour être raccordé audit corps principal (10),
**caractérisé en ce que** ledit moyen d'indication de position (60) est raccordé audit corps principal (10) le long de la direction longitudinale (A) dudit élément en saillie (4), cette dernière étant une direction préférentielle d'insertion dudit élément en saillie (4) dans l'apophyse épineuse vertébrale (50),
de sorte que ledit moyen d'indication de position (60) indique le point de pénétration dudit élément en saillie (4) dans l'apophyse épineuse vertébrale (50).

2. Dispositif chirurgical (100) selon la revendication 1, dans lequel ledit élément de contact cutané (1) a une conformation en forme de plaque, ladite conformation étant de préférence quadrangulaire ou carrée en plan.

3. Dispositif chirurgical (100) selon la revendication 1 ou 2, dans lequel ladite surface de butée (15) a un développement sensiblement plat ou planaire.

4. Dispositif chirurgical (100) selon l'une quelconque des revendications précédentes, dans lequel ledit élément de tête (30) est raccordé à ladite face supérieure (11), de préférence d'une manière permanente.

5. Dispositif chirurgical (100) selon l'une quelconque des revendications précédentes, dans lequel ledit élément de tête (30) est formé comme un écrou hexagonal.

6. Dispositif chirurgical (100) selon l'une quelconque des revendications précédentes, dans lequel ledit élément de tête (30) comprend une surface supérieure externe concave (31).

7. Dispositif chirurgical (100) selon l'une quelconque des revendications précédentes, dans lequel ledit au moins un élément en saillie (4) a une conformation globale de forme oblongue ou en forme de tige.

8. Dispositif chirurgical (100) selon l'une quelconque des revendications précédentes, dans lequel ledit au moins un élément en saillie (4) s'étend selon une direction longitudinale (A) sensiblement orthogonale par rapport à ladite surface de butée (15).

9. Dispositif chirurgical (100) selon l'une quelconque des revendications précédentes, dans lequel ledit au moins un élément en saillie (4) a une extrémité terminale libre (40) ayant une pointe pointue.

10. Dispositif chirurgical (100) selon l'une quelconque des revendications précédentes, dans lequel ledit au moins un élément en saillie (4) comprend une surface externe filetée, qui est sensiblement configurée comme une tige de vis.

11. Dispositif chirurgical (100) selon l'une quelconque des revendications précédentes, comprenant un seul élément en saillie (4) ayant un axe de symétrie coïncidant avec ladite direction longitudinale (A).

12. Dispositif chirurgical (100) selon l'une quelconque des revendications précédentes, dans lequel ledit moyen d'indication de position (60) est agencé dans une position centrée par rapport audit corps principal (10).

13. Dispositif chirurgical (100) selon l'une quelconque des revendications précédentes, ayant un axe de symétrie centrale qui coïncide avec ladite direction longitudinale (A) et définit une direction d'insertion préférentielle dudit moyen de couplage (2) dans l'apophyse épineuse vertébrale (50).

14. Dispositif chirurgical (100) selon l'une quelconque des revendications précédentes, dans lequel ledit moyen d'indication de position (60) comprend un élément de bouchon (6) et ledit élément de tête (30) comprend au moins un trou (5) obtenu au niveau de sa propre position centrale et dans ladite direction longitudinale (A), ledit élément de bouchon (6) étant configuré pour se mettre en prise, de manière réversible, avec ledit au moins un trou (5).
